# EUROPEAN PATENT APPLICATION

(11) **EP 1 611 902 A1**
(43) Date of publication of application: **04.01.2006**
(21) Application number: 04725174.9
(22) Date of filing: 01.04.2004
(51) Int. Cl.: A61K 45/00, A61K 31/4178, A61K 31/496, A61K 31/4025, A61K 31/4523, A61K 31/4164, A61K 31/135, A61P 25/04

(54) **PREVENTIVE AND/OR THERAPEUTIC AGENT FOR NEUROPATHIC PAIN**

(30) Priority: 03.04.2003 JP 2003099785
(71) Applicant: KYOWA HAKKO KOGYO CO., LTD., Chiyoda-ku, Tokyo 100-8185 (JP)
(72) Inventor: SHIRAKURA, Shiro c/o Kyowa Hakko Kogyo Co., Ltd, Shizuoka 411-8731 (JP); KUNORI, Shunji c/o Kyowa Hakko Kogyo Co., Ltd.,, Shizuoka 411-8731 (JP); TSUKII, Katsuyoshi c/o Kyowa Hakko Kogyo Co., Ltd., Shizuoka 411-8731 (JP); KATAYAMA, Keishi Kyowa Hakko Kogyo Co., Ltd.,, Shizuoka 411-8731 (JP); TOKI, Shinichiro c/o Kyowa Hakko Kogyo Co., Ltd.,, Shizuoka 411-8731 (JP); HIROSE, Ryo Kyowa Hakko Kogyo Co., Ltd.,, Shizuoka 411-8731 (JP)
(74) Representative: Tanner, James Percival
(86) International application number: PCT/JP2004/004758
(87) International publication number: WO 2004/089410

(57) **Abstract**

A preventive and/or therapeutic agent for neuropathic pain which comprises, as an active ingredient, a compound having histamine H3-receptor antagonism such as a compound represented by Formula (II): 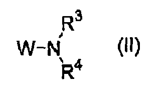
(wherein W represents a residue which imparts antagonistic and/or agonistic activity at histamine H3-receptors when attached to an imidazole ring in 4- or 5-position,
and R³ and R⁴ may be the same or different and each represents substituted or unsubstituted lower alkyl or the like) or a pharmaceutically acceptable salt thereof is provided.

## Description

### Technical Field

The present invention relates to a preventive and/or therapeutic agent for neuropathic pain which comprises, as an active ingredient, a compound having histamine H3-receptor antagonism.

### Background Art

Neuropathic pain is pathological intractable pain caused by a dysfunction of the peripheral or central nervous system, which is contrast to sensations and acute pain which occur as biological warning signals for protecting living bodies from injuries and harms. It is known that neuropathic pain is caused by neural damage resulting from an injury, a surgery, an infection with the herpesvirus, human immunodeficiency virus or the like, cancer, a metabolic disorders such as diabetes, or the like. Although the mechanisms underlying neuropathic pain are poorly understood, it is thought to be a result of functional and plastic changes in the peripheral or central nervous system. The neuropathic pain is treated by a nerve block therapy, an electric stimulation therapy, or a medical therapy with an antiepileptic, an anticonvulsant, a non-steroidal antiinflammatory agent, or the like. However, complete relief is rarely achieved excluding the effect of carbamazepine on trigeminal neuralgia. Hence, the development of more effective therapeutic agents for neuropathic pain is expected.

On the other hand, it is known that there are the four distinct subtypes of histamine receptors, H1, H2, H3 and H4. The histamine H3-receptor was found by Arrang et al in 1983 (Nature, 1983, Vol. 302, pp. 832-837), and cloned in 1999 (Molecular Pharmacology, 1999, Vol. 55, pp. 1101-1107). The histamine H3-receptor is expressed in the sensory neurons, the spinal cords, and the central nervous system, and the histamine H3-receptor functions as an auto-receptor or hetero-receptor for regulating the release of neurotransmitters. As diseases to which histamine H3-receptor antagonists are applied, an Alzheimer's disease, an eating disorder, an insomnia, an attention-deficit hyperactivity disorder, and the like have been assumed (EP09832300). It has been reported that a histamine H3-receptor antagonist is involved in the modulation of acute nociceptive pain (for example, British Journal of Pharmacology, 1994, Vol. 111, pp. 1269-1279 and Pharmacology, Biochemistry and Behavior, 2002, Vol. 72, pp. 751-760). However, to date, a relationship between a histamine H3-receptor antagonist and neuropathic pain has not been reported.

### Disclosure of Invention

An object of the present invention is to provide a preventive and/or therapeutic agent for neuropathic pain which comprises, as an active ingredient, a compound having histamine H3-receptor antagonism.

The present invention relates to the following (1) to (24) :
(1) A preventive and/or therapeutic agent for neuropathic pain which comprises, as an active ingredient, a compound having histamine H3-receptor antagonism or a pharmaceutically acceptable salt thereof.
(2) The preventive and/or therapeutic agent for neuropathic pain according to (1), wherein the compound having histamine H3-receptor antagonism is a compound represented by Formula (I) : 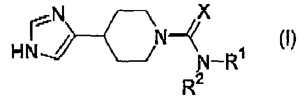
   (wherein R¹ and R² may be the same or different and each represents a hydrogen atom, substituted or unsubstituted lower alkyl or substituted or unsubstituted cycloalkyl, or R¹ and R² are combined together with the adjacent nitrogen atom thereto to form a substituted or unsubstituted heterocyclic group;
   and X represents an oxygen atom or a sulfur atom).
(3) The preventive and/or therapeutic agent for neuropathic pain according to (2), wherein one of R¹ and R² is a hydrogen atom, and the other is substituted or unsubstituted lower alkyl or substituted or unsubstituted cycloalkyl, and X is a sulfur atom.
(4) The preventive and/or therapeutic agent for neuropathic pain according to (1), wherein the compound having histamine H3-receptor antagonism is thioperamide.
(5) The preventive and/or therapeutic agent for neuropathic pain according to (1), wherein the compound having histamine H3-receptor antagonism is a compound represented by Formula (II): 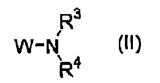
   (wherein W represents a residue which imparts antagonistic and/or agonistic activity at histamine H3-receptors when attached to an imidazole ring in 4- or 5-position, and R³ and R⁴ may be the same or different and each represents substituted or unsubstituted lower alkyl or substituted or unsubstituted cycloalkyl, or R³ and R⁴ are combined together with the adjacent nitrogen atom thereto to form a substituted or unsubstituted heterocyclic group) or a pharmaceutically acceptable salt thereof.
(6) The preventive and/or therapeutic agent for neuropathic pain according to (5), wherein R³ and R⁴ may be the same or different and each is substituted or unsubstituted lower alkyl or substituted or unsubstituted cycloalkyl.
(7) The preventive and/or therapeutic agent for neuropathic pain according to (5), wherein R³ and R⁴ are combined together with the adjacent nitrogen atom thereto to form a substituted or unsubstituted nitrogen-containing heterocyclic group.
(8) The preventive and/or therapeutic agent for neuropathic pain according to (5), wherein -NR³R⁴ is a group represented by Formula (III): 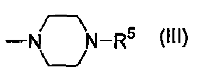
   (wherein R⁵ represents lower alkyl, cycloalkyl, aryl, aralkyl, lower alkanoyl, cycloalkanoyl, aroyl, lower alkoxycarbonyl or aminoalkylcarbonyl).
(9) The preventive and/or therapeutic agent for neuropathic pain according to any of (5) to (8), wherein W is a group represented by Formula (IV): 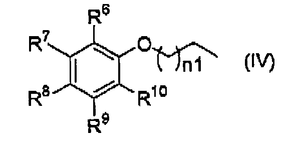
   (wherein R⁶, R⁷, R⁸, R⁹ and R¹⁰ may be the same or different and each represents a hydrogen atom, halogen, amino, nitro, cyano, substituted or unsubstituted lower alkyl, substituted or unsubstituted lower alkoxy, substituted or unsubstituted lower alkanoyl, substituted or unsubstituted cycloalkanoyl, substituted or unsubstituted aroyl or substituted or unsubstituted lower alkanoylamino, and n1 represents an integer of 1 to 7).
(10) The preventive and/or therapeutic agent for neuropathic pain according to any of (5) to (8), wherein W is a group represented by Formula (V): 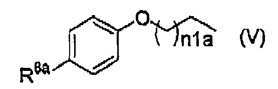
   (wherein R^{8a} has the same meaning as R⁸ defined above, and nla has the same meaning as n1 defined above).
(11) The preventive and/or therapeutic agent for neuropathic pain according to (10), wherein n1 is 1 or 2.
(12) The preventive and/or therapeutic agent for neuropathic pain according to (1), wherein the compound having histamine H3-receptor antagonism is a compound represented by Formula (VI): 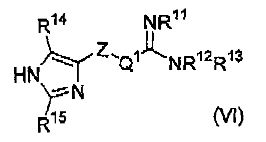
   [wherein Z represents substituted or unsubstituted lower alkylene;
   Q¹ represents a sulfur atom, -NH- or -CH₂-;
   R¹¹, R¹³ and R¹⁵ may be the same or different and each represents a hydrogen atom, lower alkyl, cycloalkyl, substituted or unsubstituted aryl or substituted or unsubstituted aralkyl;
   R¹² represents a hydrogen atom, lower alkyl, (cycloalkyl)alkyl, substituted or unsubstituted aryl, substituted or unsubstituted aralkyl or a group represented by Formula (VII) : 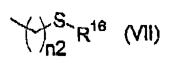
   (wherein n2 represents an integer of 1 to 4, and R¹⁶ represents lower alkyl, (cycloalkyl)alkyl or substituted or unsubstituted aralkyl);
   and R¹⁴ represents a hydrogen atom, halogen, amino, nitro, cyano, substituted or unsubstituted lower alkyl, substituted or unsubstituted aryl or substituted or unsubstituted aralkyl] or a pharmaceutically acceptable salt thereof.
(13) The preventive and/or therapeutic agent for neuropathic pain according to (1), wherein the compound having histamine H3-receptor antagonism is clobenpropit.
(14) The preventive and/or therapeutic agent for neuropathic pain according to (1), wherein the compound having histamine H3-receptor antagonism is a compound represented by Formula (VIII) : 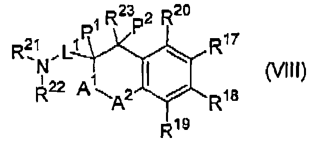
   {wherein A¹ represents a bond or carbonyl;
   A² represents an oxygen atom or a sulfur atom;
   L¹ represents lower alkylene which may be substituted by a fluorine atom or hydroxy;
   P¹ and P² represent hydrogen atoms or are combined to represent a bond;
   R¹⁷, R¹⁸, R¹⁹ and R²⁰ may be the same or different and each represents a hydrogen atom, halogen, nitro, hydroxy, mercapto, cyano, carboxy, lower alkanoyl, lower alkoxycarbonyl, lower alkanoyloxy, lower alkylthio, lower alkylsulfinyl, lower alkylsulfonyl, substituted or unsubstituted lower alkyl, substituted or unsubstituted cycloalkyl, substituted or unsubstituted lower alkoxy, substituted or unsubstituted aryl, a substituted or unsubstituted heterocyclic group, -NR^{24a}R^{24b} (wherein R^{24a} and R^{24b} may be the same or different and each represents a hydrogen atom, lower alkyl or lower alkanoyl),
   -C(=O)-NR^{24c}R^{24d} (wherein R^{24c} and R^{24d} have the same meanings as R^{24a} and R^{24b} defined above, respectively), -SO₂-NR^{24e}R^{24f} (wherein R^{24e} and R^{24f} have the same meanings as R^{24a} and R^{24b} defined above, respectively), -L²-R²⁵ wherein L² represents an oxygen atom, a sulfur atom, lower alkylene, lower alkenylene, -S(O)-, -S(O)₂- -C(O)-, -C(=NOR²⁶)- (wherein R²⁶ represents a hydrogen atom or lower alkyl) or -N(R²⁷)-(wherein R²⁷ represents a hydrogen atom, lower alkyl or lower alkanoyl), and R²⁵ represents cycloalkyl, aryl or a heterocyclic group], or -L³-L⁴-R²⁸ [wherein L³ represents cycloalkylene, arylene, a divalent group formed by removing any one hydrogen atom from an aliphatic heterocyclic group or heteroarylene, L⁴ represents a bond, an oxygen atom, a sulfur atom, lower alkylene, lower alkenylene, -C(O)-, -C(=NOR^{26a})- (wherein R^{26a} has the same meaning as R²⁶ defined above) or -N(R^{27a})- (wherein R^{27a} has the same meaning as R²⁷ defined above), and R²⁸ has the same meaning as R²⁵ defined above]; at least one of R¹⁷, R¹⁸, R¹⁹ and R²⁰ represents cycloalkyl, substituted or unsubstituted aryl, a substituted or unsubstituted heterocyclic group, -L²-R²⁵ (wherein L² and R²⁵ have the same meanings as defined above, respectively) or -L³-L⁴-R²⁸ (wherein L³, L⁴ and R²⁸ have the same meanings as defined above, respectively);
   R²¹ and R²² may be the same or different and each represents a hydrogen atom, lower alkyl, hydroxy-lower alkyl, cycloalkyl, (cycloalkyl)alkyl, lower alkenyl, lower alkynyl, aryl, aralkyl, a heterocyclic group or heterocyclic alkyl, or R²¹ and R²² are combined together with the adjacent nitrogen atom thereto to form a substituted or unsubstituted nitrogen-containing heterocyclic group;
   and R²³ represents a hydrogen atom, halogen, nitro, hydroxy, mercapto, cyano, carboxy, lower alkoxycarbonyl, lower alkanoyl, lower alkanoyloxy, lower alkylsulfinyl, lower alkylsulfonyl, lower alkylthio, aryl, a heterocyclic group, substituted or unsubstituted lower alkyl, substituted or unsubstituted lower alkoxy, -NR^{29a}R^{29b} (wherein R^{29a} and R^{29b} have the same meanings as R^{24a} and R^{24b} defined above, respectively), -C(=O)-NR^{29c}R^{29d} (wherein R^{29c} and R^{29d} have the same meanings as R^{24a} and R^{24b} defined above, respectively) or -SO₂-NR^{29e}R^{29f} (wherein R^{29e} and R^{29f} have the same meanings as R^{24a} and R^{24b} defined above, respectively)}.
(15) The preventive and/or therapeutic agent for neuropathic pain according to (1), wherein the compound having histamine H3-receptor antagonism is a compound (ABT-239) represented by Formula (VIIIa): 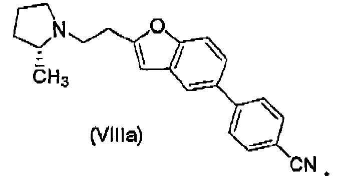
(16) The preventive and/or therapeutic agent for neuropathic pain according to (1), wherein the compound having histamine H3-receptor antagonism is a compound represented by Formula (IX): 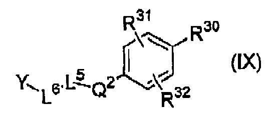
   {wherein Y represents a group represented by Formula (X): 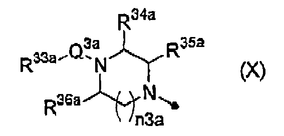
   [wherein n3a is 1 or 2;
   Q^{3a} represents a bond, -C(O)-, -C(S)-, -CH₂-, -SO₂- or
   -C(=NR³⁷)- (wherein R³⁷ represents a hydrogen atom, hydroxy, lower alkyl, cycloalkyl, (cycloalkyl)alkyl, lower alkoxy, aryl or aralkyl);
   R^{33a} represents a hydrogen atom, amino, lower alkyl, lower alkoxy, cycloalkoxy, substituted or unsubstituted cycloalkyl, substituted or unsubstituted aryl, substituted or unsubstituted aryloxy, a substituted or unsubstituted heterocyclic group or -W¹-C(R^{38a}) (R^{38b})-NR^{39a}R^{39b} (wherein W¹ represents a bond or substituted or unsubstituted lower alkylene, R^{38a} and R^{38b} may be the same or different and each represents a hydrogen atom, amino, aralkyl or substituted or unsubstituted lower alkyl, and R^{39a} and R^{39b} may be the same or different and each represents a hydrogen atom, aminosulfonyl, lower alkyl, lower alkanoyl, lower alkylsulfonyl, cycloalkyl, (cycloalkyl)alkyl, cycloalkanoyl, cycloalkylsulfonyl, aralkyl, aroyl, arylsulfonyl, heterocyclic alkyl, heterocyclic carbonyl, heterocyclic alkanoyl, heterocyclic sulfonyl, substituted or unsubstituted aryl, a substituted or unsubstituted aromatic heterocyclic group or a substituted or unsubstituted aliphatic heterocyclic group, or R^{39a} and R^{39b} are combined together with the adjacent nitrogen atom thereto to form a substituted or unsubstituted nitrogen-containing heterocyclic group, or R^{38a} or R^{38b} and R^{39a} or R^{39b} are combined together with the adjacent carbon atom and nitrogen atom thereto to form a substituted or unsubstituted heterocyclic group);
   and R^{34a}, R^{35a} and R^{36a} may be the same or different and each represents a hydrogen atom or lower alkyl], a group represented by Formula (XI): 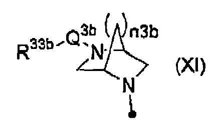
   (wherein n3b, Q^{3b} and R^{33b} have the same meanings as n3a, Q^{3a} and R^{33a} defined above, respectively), a group represented by Formula (XII): 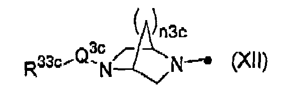
   (wherein n3c, Q^{3c} and R^{33c} have the same meanings as n3a, Q^{3a} and R^{33a} defined above, respectively) or a group represented by Formula (XIII): 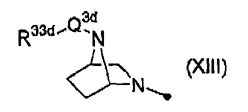
   (wherein Q^{3d} and R^{33d} have the same meanings as Q^{3a} and R^{33a} defined above, respectively);
   L⁵ represents a bond or lower alkylene which may be substituted by substituted or unsubstituted aryl;
   L⁶ represents a bond, substituted or unsubstituted lower alkylene, or substituted or unsubstituted cycloalkylene, and
   L⁵ and L⁶ do not represent bonds simultaneously;
   Q² represents an oxygen atom, a sulfur atom, -S(O)-, -S(O)₂- or -C≡C-;
   R³⁰ represents halogen, amino, cyano, aminocarbonyl, cycloalkyl, lower alkoxy, lower alkanoyl, cycloalkanoyl, lower alkoxycarbonyl, mono- or di(lower alkyl)aminocarbonyl, aralkyl, aroyl, arylsulfonyl, aromatic heterocyclic carbonyl, aromatic heterocyclic sulfonyl, substituted or unsubstituted lower alkyl, substituted or unsubstituted aryl, a substituted or unsubstituted aromatic heterocyclic group, -CHR^{40a}-OR^{41a} (wherein R^{40a} represents a hydrogen atom, lower alkyl, cycloalkyl, (cycloalkyl)alkyl, aryl or aralkyl, and R^{41a} represents a hydrogen atom, lower alkyl, cycloalkyl, (cycloalkyl)alkyl, lower alkanoyl, lower alkoxycarbonyl, tri (lower alkyl) silyl, aryl or aralkyl), or -C(R^{40b})=N-OR^{41b} (wherein R^{40b} and R^{41b} have the same meanings as R^{40a} and R^{41a} defined above, respectively);
   and R³¹ and R³² may be the same or different and each represents a hydrogen atom, halogen, amino, nitro, azido, hydroxy, cyano, formyl, carboxy, lower alkyl, perfluoro lower alkyl, lower alkenyl, lower alkynyl, lower alkoxy or perfluoro lower alkoxy, or R³¹ and R³² are combined to represent -OCH₂C(O)-}.
(17) The preventive and/or therapeutic agent for neuropathic pain according to (1), wherein the compound having histamine H3-receptor antagonism is a compound (A-304121) represented by Formula (IXa): 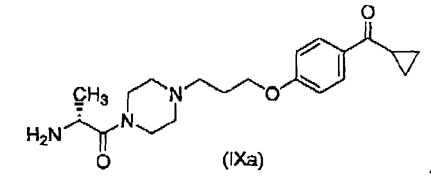
(18) The preventive and/or therapeutic agent for neuropathic pain according to (1), wherein the compound having histamine H3-receptor antagonism is a compound (A-317920) represented by Formula (IXb): 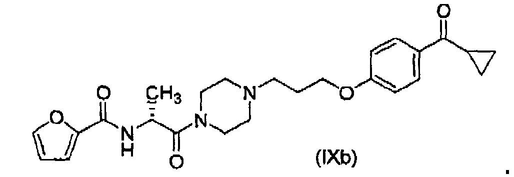
(19) The preventive and/or therapeutic agent for neuropathic pain according to (1), wherein the compound having histamine H3-receptor antagonism is a compound represented by Formula (XIV): 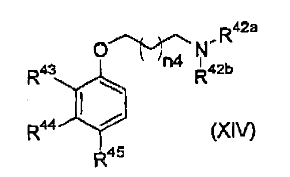
   {wherein n4 represents an integer of 0 to 4;
   R^{42a} and R^{42b} may be the same or different and each represents lower alkyl, lower alkenyl, cycloalkyl or (cycloalkyl)alkyl, or R^{42a} and R^{42b} are combined together with the adjacent nitrogen atom thereto to form a nitrogen-containing heterocyclic group;
   and two of R⁴³, R⁴⁴ and R⁴⁵ may be the same or different and each represents a hydrogen atom or halogen, and the remainder represents a substituted or unsubstituted heterocyclic group, substituted or unsubstituted heterocyclic alkyl, substituted or unsubstituted heterocyclic alkenyl, substituted or unsubstituted heterocyclic alkynyl, -L⁷-L⁸-Q⁴ [ wherein L⁷ represents a bond or an oxygen atom, L⁸ represents substituted or unsubstituted lower alkylene, cycloalkylene, alkenylene or alkynylene, and Q⁴ represents an aliphatic heterocyclic group or -NR^{46a}R^{46b} (wherein R^{46a} and R^{46b} may be the same or different and each represents a hydrogen atom, lower alkyl, lower alkenyl, cycloalkyl, (cycloalkyl)alkyl, aryl, aralkyl, a heterocyclic group or heterocyclic alkyl)], -N(L^{8a}-Q^{4a})R⁴⁷ (wherein L^{8a} and Q^{4a} have the same meanings as L⁸ and Q⁴ defined above, respectively, and R⁴⁷ represents a hydrogen atom, lower alkyl, lower alkenyl, cycloalkyl, (cycloalkyl)alkyl, a heterocyclic group or heterocyclic alkyl) or -L⁹-C(L^{8b}-Q^{4b})R⁴⁸R⁴⁹ (wherein L^{8b} and Q^{4b} have the same meanings as L⁸ and Q⁴ defined above, respectively, L⁹ represents a bond, substituted or unsubstituted lower alkylene, cycloalkylene, alkenylene or alkynylene, R⁴⁸ represents a hydrogen atom, lower alkyl, lower alkenyl, cycloalkyl, (cycloalkyl)alkyl, a heterocyclic group or heterocyclic alkyl, and R⁴⁹ represents a hydrogen atom, halogen, hydroxy or lower alkoxy)}.
(20) The preventive and/or therapeutic agent for neuropathic pain according to (1), wherein the compound having histamine H3-receptor antagonism is a compound (JNJ-5207852) represented by Formula (XIVa): 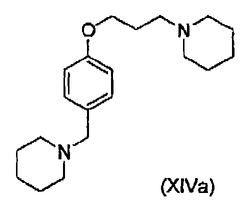
(21) A method for preventing and/or treating neuropathic pain, which comprises administering an effective amount of the compound having histamine H3-receptor antagonism.
(22) The method for preventing and/or treating neuropathic pain according to (21), wherein the compound having histamine H3-receptor antagonism is the compound described in any of (2) to (20).
(23) Use of a compound having histamine H3-receptor antagonism, for the manufacture of a preventive and/or therapeutic agent for neuropathic pain.
(24) The use of a compound having histamine H3-receptor antagonism according to (23), wherein the compound having histamine H3-receptor antagonism is the compound described in any of (2) to (20).

A compound represented by Formula (I), (II), (VI), (VIII), (IX) and (XIV) is referred to as Compound (I), (II), (VI), (VIII), (IX) and (XIV) hereinafter. Compounds represented by other Formula numbers are also referred to in the same manner.

In the definition of each group in Formula (I) to (XIV), examples described below are mentioned.

Examples of the halogen include fluorine, chlorine, bromine and iodine atoms.

Examples of the lower alkyl include linear or branched alkyl having 1 to 10 carbon atoms, e.g. methyl, ethyl, propyl, isopropyl, butyl, isobutyl, sec-butyl, tert-butyl, pentyl, isopentyl, neopentyl, hexyl, heptyl, octyl, isooctyl, nonyl, decyl.

The lower alkyl moiety of the lower alkoxy, the lower alkoxycarbonyl, the lower alkylthio, the lower alkyl sulfinyl, the lower alkylsulfonyl, the mono or di(lower alkyl)aminocarbonyl and the tri(lower alkyl)silyl have the same meanings as the lower alkyl defined above, respectively. Two lower alkyl moieties of the di(lower alkyl)aminocarbonyl and three lower alkyl moieties of the tri(lower alkyl)silyl may be the same or different.

The lower alkylene and the alkylene moiety of the hydroxy-lower alkyl, the (cycloalkyl)alkyl, the aminoalkylcarbonyl, the aralkyl and the heterocyclic alkyl have the same meanings as the groups formed by removing one hydrogen atom from the lower alkyl defined above.

Examples of the cycloalkyl include cycloalkyl having 3 to 8 carbon atoms, e.g. cyclopropyl, cyclobutyl, cyclopentyl, cyclohexyl, cycloheptyl, cyclooctyl.

The cycloalkyl moiety of the (cycloalkyl)alkyl, the cycloalkoxy, the lower cycloalkanoyl and the cycloalkylsulfonyl have the same meanings as the cycloalkyl defined above.

The cycloalkylene has the same meaning as the group formed by removing one hydrogen atom from the cycloalkyl defined above.

Examples of the lower alkenyl include linear or branched alkenyl having 2 to 6 carbon atoms, e.g. vinyl, allyl, 1-propenyl, methacryl, crotyl, 1-butenyl, 3-butenyl, 2-pentenyl, 4-pentenyl, 2-hexenyl, 5-hexenyl.

The lower alkenylene and the alkenylene moiety of the heterocyclic alkenyl have the same meanings as the groups formed by removing one hydrogen atom from the lower alkenyl defined above.

Examples of the lower alkynyl include linear or branched alkynyl having 2 to 6 carbon atoms, e.g. ethynyl, propynyl, butynyl, pentynyl, hexynyl.

The lower alkynylene and the alkynylene moiety of the heterocyclic alkynyl have the same meanings as the groups formed by removing one hydrogen atom from the lower alkynyl defined above.

Examples of the lower alkanoyl and the lower alkanoyl moieties of the lower alkanoylamino and the lower alkanoyloxy include linear or branched alkanoyl having 1 to 7 carbon atoms, e.g. formyl, acetyl, propionyl, butyryl, isobutyryl, valeryl, isovaleryl, pivaloyl, hexanoyl, heptanoyl.

The alkylenecarbonyl moiety of the heterocyclic alkanoyl has the same meaning as the group formed by removing one hydrogen atom from the lower alkanoyl defined above.

Examples of the aryl include aryl having 6 to 14 carbon atoms, e.g. phenyl, naphthyl, anthryl.

The aryl moieties of the aralkyl, the aryloxy, the aroyl and the arylsulfonyl have the same meanings as the aryl defined above.

The arylene has the same meaning as the group formed by removing one hydrogen atom from the aryl defined above.

Examples of the heterocyclic group include an aromatic heterocyclic group and an aliphatic heterocyclic group.

The heterocyclic moieties of the heterocyclic alkyl, the heterocyclic alkenyl, the heterocyclic alkynyl, the heterocyclic carbonyl, the heterocyclic alkanoyl and the heterocyclic sulfonyl have the same meanings as the heterocyclic group defined above.

Examples of the aromatic heterocyclic group include a 5- or 6-membered monocyclic group containing at least one atom selected from an nitrogen atom, an oxygen atom and a sulfur atom, and bicyclic or tricyclic condensed aromatic heterocyclic group which is formed by condensation of 3- to 8-membered rings and contains at least one atom selected from a nitrogen atom, an oxygen atom, and a sulfur atom. Specific examples include pyridyl, pyridonyl, pyrazinyl, pyrimidinyl, pyridazinyl, quinolyl, isoquinolyl, phthalazinyl, quinazolinyl, quinoxalinyl, naphthyridinyl, cinnolinyl, pyrrolyl, pyrazolyl, imidazolyl, triazolyl, tetrazolyl, thienyl, furyl, thiazolyl, oxazolyl, indolyl, indazolyl, benzimidazolyl, isoxazolyl, benzotriazolyl, benzothiazolyl, benzoxazolyl, purinyl, and the like.

The heteroaromatic moieties of the heteroaromatic carbonyl and the heteroaromatic sulfonyl have the same meanings as the aromatic heterocyclic group defined above.

The heteroarylene has the same meaning as the group formed by removing one hydrogen atom from the aromatic heterocyclic group defined above.

Examples of the aliphatic heterocyclic group include a 5- or 6-membered monocyclic heterocyclic group containing at least one atom selected from a nitrogen atom, an oxygen atom, and a sulfur atom, and a bicyclic or tricyclic condensed heterocyclic group which is formed by condensation 3- to 8-membered rings and which contains at least one atom selected from a nitrogen atom, an oxygen atom, and a sulfur atom. Specific examples include pyrrolidinyl, pyrrolidonyl, piperidino, piperidyl, piperazinyl, morpholino, morpholinyl, thiomorpholino, thiomorpholinyl, homopiperidino, homopiperidyl, homopiperazinyl, tetrahydropyridyl, tetrahydroquinoyl, tetrahydroisoquinoyl, tetrahydrofuranyl, tetrahydropyranyl, dihydrobenzofuranyl, and the like.

The aliphatic heterocyclic moiety of the divalent group formed by removing one hydrogen atom from an aliphatic heterocyclic group has the same meaning as the aliphatic heterocyclic group defined above.

Examples of the nitrogen containing-heterocyclic group formed together with the adjacent nitrogen atom and the heterocyclic group formed together with the adjacent carbon atom and nitrogen atom include a 3- to 9-membered monocyclic heterocyclic group containing at least one nitrogen atom (the monocyclic heterocyclic group may contain other nitrogen atoms, oxygen atoms or sulfur atoms), a bicyclic or tricyclic condensed heterocyclic group which is formed by condensation 3- to 8-membered rings and which contains at least one nitrogen atom (the condensed heterocyclic group may contain other nitrogen atoms, oxygen atoms or sulfur atoms). Specific examples include aziridinyl, pyrrolidinyl, piperidino, piperazinyl, morpholino, thiomorpholino, homopiperidino, homopiperazinyl, tetrahydropyridinyl, tetrahydroquinolyl, tetrahydroisoquinolyl, perhydroazocinyl, and the like.

The substituents of the substituted lower alkyl, the substituted lower alkylene, the substituted cycloalkyl, the substituted cycloalkylene, the substituted lower alkoxy, the substituted lower alkanoyl, the substituted cycloalkanoyl, the substituted aryl, the substituted aralkyl, the substituted aryloxy, the substituted aroyl, the substituted lower alkanoylamino, the substituted heterocyclic group, the substituted heterocyclic alkyl, the substituted heterocyclic alkenyl, the substituted heterocyclic alkynyl, the substituted aromatic heterocyclic group, the substituted aliphatic heterocyclic group, the substituted nitrogen containing-heterocyclic group formed with the adjacent nitrogen atom and the substituted heterocyclic group formed with the adjacent carbon atom and nitrogen atom, may be the same or different. The number of substituents is 1 to 8, and examples of the substituents include halogen, amino, azido, nitro, hydroxy, mercapto, cyano, carboxy, lower alkyl, cycloalkyl, aryl, aralkyl, lower alkanoyl, cycloalkanoyl, aroyl, lower alkoxycarbonyl, aminoalkylcarbonyl, and the like. Here, the halogen, the lower alkyl, the cycloalkyl, the aryl, the lower alkanoyl and the lower alkoxycarbonyl have the same meanings as defined above, respectively. The cycloalkyl moiety of the cycloalkanoyl has the same meaning as defined above. The alkylene moieties of the aralkyl and the aminoalkylcarbonyl have the same meanings as defined above, respectively. The aryl moieties of the aralkyl and the aroyl have the same meanings as defined above, respectively.

The perfluoro lower alkyl and the perfluoro lower alkoxy are lower alkyl and lower alkoxy whose all hydrogen atoms are substituted by fluorine atoms, respectively.

W may be any residue which imparts antagonistic and/or agonistic activity at histamine H3-receptors when attached to an imidazole ring in 4- or 5-position. Example of such residue include a group represented by Formula (IV): 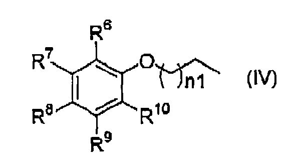
(wherein R⁶, R⁷, R⁸, R⁹, R¹⁰ and n1 have the same meanings as defined above, respectively).

The pharmaceutically acceptable salts of Compound (I), (II), (VI), (VIII), (IX) and (XIV) are, for example, pharmaceutically acceptable acid addition salts, metal salts, ammonium salts, organic amine addition salts, and amino acid addition salts. Examples of the pharmaceutically acceptable acid addition salts include inorganic acid salts, e.g. hydrochloride, sulfate, nitrate and phosphate; and organic acid salts, e.g. acetate, maleate, fumarate, and citrate. Examples of the pharmaceutically acceptable metal salts include alkali-metal salts, e.g. sodium salt and potassium salt; alkaline-earth metal salts, e.g. magnesium salts and calcium salts; aluminium salts, and zinc salts. Examples of the pharmaceutically acceptable ammonium salts include ammonium salts and tetramethylammonium salts. Examples of the pharmaceutically acceptable organic amine addition salts include addition salts of morpholine and piperidine. Examples of the pharmaceutically acceptable amino acid addition salts include addition salts of glycine, phenylalanine, lysine, aspartic acid, and glutamic acid.

The compound having histamine H3-receptor antagonism which is used as the active ingredient of the preventive and/or therapeutic agent for neuropathic pain of the present invention may have any structures as long as they have histamine H3-receptor antagonism. For example, the compounds may be peptide compounds or nonpeptide compounds.

The neuropathic pain is chronic intractable pain caused by some functional disorder which results from damage in the peripheral or central nervous system due to an injury, compression, surgery, infection with herpesvirus, human immunodeficiency virus or the like, or a disease such as a cancer, a metabolism disorder including diabetes, or the like. Neuropathic pain includes entrapment neuropathy, phantom limb pain, postoperative neuralgia, postherpetic neuralgia, postapoplectic thalamic pain, neuropathic low back pain, a spinal cord injury, trigeminal neuralgia, glossopharyngeal neuralgia, and cancer pain which is less responsive to opioids therapy such as morphine or the like. Typical symptoms include, but are not limited to, burning pain, allodynia (feeling a severe pain in response to a non-nociceptive stimulus), and hyperalgesia.

Examples of the compound having histamine H3-receptor antagonism include the compounds described below:
Thioperamide, cipralist, GT-2227, GT-2394, A-331440, A-317920, A-304121, A-320436, ABT-239, ABT-834, UCL-1972, UCL-1409, FUB-181, FUB-470, Sch-50971, impentamine, ciproxifan, clobenpropit, JNJ-5207852, VUF-5228, and the compounds described in the documents or patents below.
Folia Pharmacological Japonica, 1999, Vol. 114, No. 2, pp. 89-106
Bioorganic and Medicinal Chemistry Letters, 2000, Vol. 10, pp. 2379-2382 and 2002, Vol. 12, pp. 3309-3312
Drugs of the Future, 1996, Vol. 21, pp. 507-520
EP0978512, EP0982300, US6008240, US6211199, US6297259, US6316475, US6329392, US2001/0049367, US2002/0035103, US2002/0111340, US2003/134835, WO92/15567, WO93/12093, WO93/14070, WO94/17058, WO95/14007, WO96/29315, WO96/38141, WO96/38142, WO97/29092, WO99/24405, WO99/31089, WO00/06254, WO00/06552, WO00/42023, WO00/63208, WO00/64884, WO01/66534, WO01/68651, WO01/68652, WO01/73023, WO01/74773, WO01/74810, WO01/74813, WO01/74814, WO01/74815, WO02/06223, WO02/12190, WO02/12214, WO02/12224, WO02/13821, WO02/15905, WO02/24657, WO02/24658, WO02/24659, WO02/24695, WO02/32893, WO02/40461, WO02/44141, WO02/056871, WO02/074758, WO02/076925, WO02/079168, WO03/040106, WO03/050099, WO03/059341, WO03/059342, WO03/064411, WO03/066604, and WO03/104235.

These compounds can be synthesized by the methods described in the literatures or patents described above.

Next, the pharmacological activities of typical compounds will be described in detail by test examples.

As neuropathic pain models, a rat with chronic constriction injury, which is a rat model of neuropathic pain resulting from sciatic nerve injury, and a rat with streptozotocin-induced diabetic pain, which is a model of neuropathic pain resulting from diabetic nerve injury (Pain, 1996, Vol. 68, pp. 293-299) were used. It has been reported that gabapentin, an anticonvulsant, which has been approved for the management of neuropathic pain (postherpetic neuralgia, neuropathic low back pain and diabetic neuralgia) in Europe, relieves pain-related behaviors in rats with chronic constriction injury and rats with streptozotocin-induced diabetes (Pain, 1999, Vol. 80, pp. 391-398 and British Journal of Pharmacology, 2000, Vol. 131, pp.282-286).

As test compounds for evaluating the neuropathic pain inhibitory action of a histamine H3-receoptor antagonist, thioperamide, Compound A, A-304121, A-317920, ABT-239, JNJ-5027852 and clobenpropit were used. Compound A is Compound 43 [N-{3-(4-cyanophenoxy)propyl}diethylamine hydrogen oxalate] disclosed in EP0982300.

### Test Example 1: Neuropathic pain inhibitory action of test compounds in rats with chronic constriction injury (1)

Experiments were conducted according to the method of G. M. Pitcher et al. (Pain, 1999, Vol. 83, pp. 37-46), which was an improvement on the method of T. Mosconi, L. Kruger, et al. (Pain, 1996, Vol. 64, pp. 37-57).

Male SD rats (Sprague-Dawley rats) were used for the experiments. Under the pentobarbital anesthesia, the sciatic nerve was exposed as near the central side as possible, and it was wrapped with a PE-60 polyethylene tube cuff (trade name: Intramedic, size: PE-60, manufactured by Becton Dickinson & Company) of 2 mm in length to prepare rats with chronic constriction injury. On day 14 to 21 after the surgery, rats were tested. Rats with 50% paw withdrawal threshold to von Frey filament (trade name: touch test sensory evaluator, model No. Model 58011, manufactured by Muromachi Kikai Co., Ltd.) less than 4 g were used for the evaluation of test compounds.

Allodynia, a typical symptom in neuropathic pain, or hyperalgesia was evaluated using von Frey filaments. Pain threshold was determined by the up down method of W. J. Dixon (Annual Review of Pharmacology and Toxicology, 1980, Vol. 20, pp. 441-462). Rats were placed in stainless cages (width 750 mm × length 210 mm × height 170 mm) and allowed to acclimate for at least 20 minutes before the test. The pain thresholds of normal rats were about 10 g, but decreases in pain threshold were observed in the ipsilateral paws of the rats with chronic constriction injury. The pain thresholds were measured before the administration of test compounds and 0.5, 1, 1.5 and 2 hour after the administration. Each test compound was suspended in distilled water and intraperitoneally administered in a volume of 2 mL/kg.

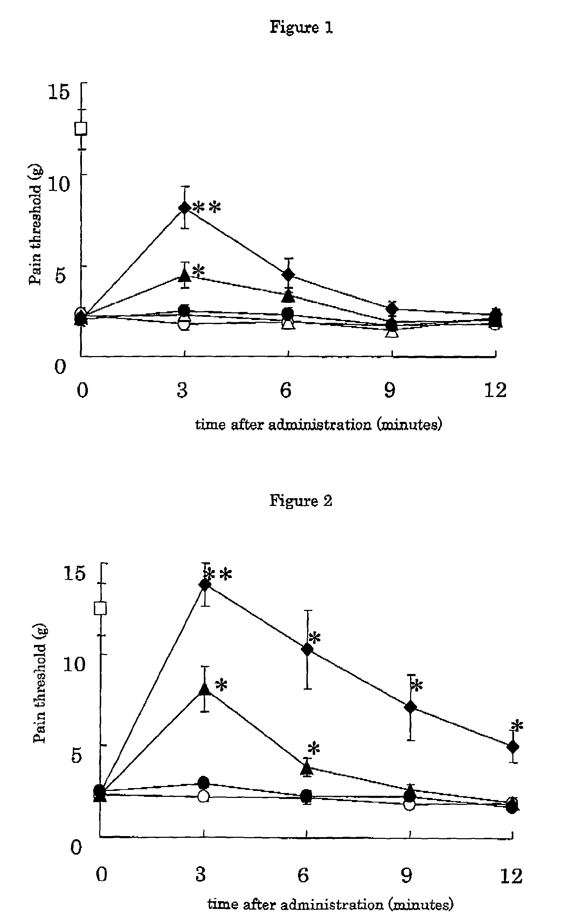

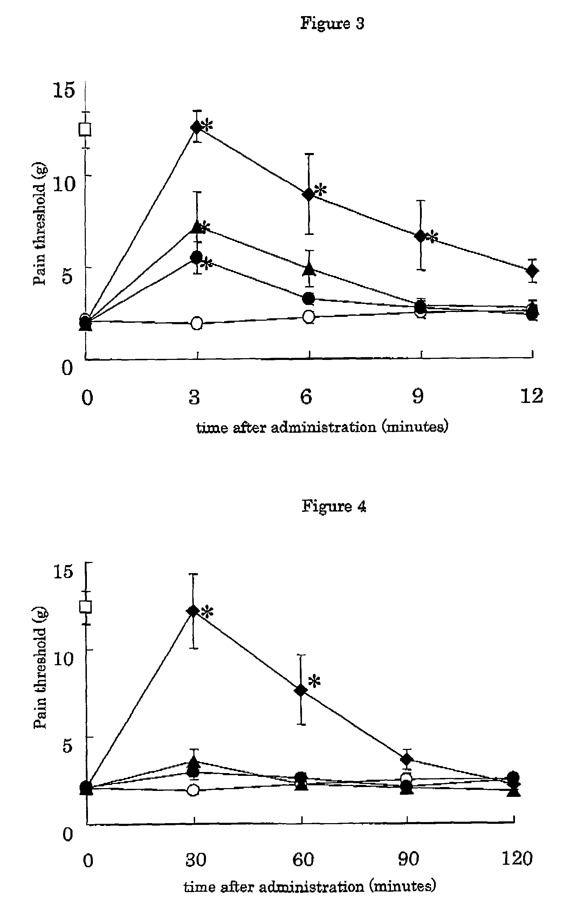

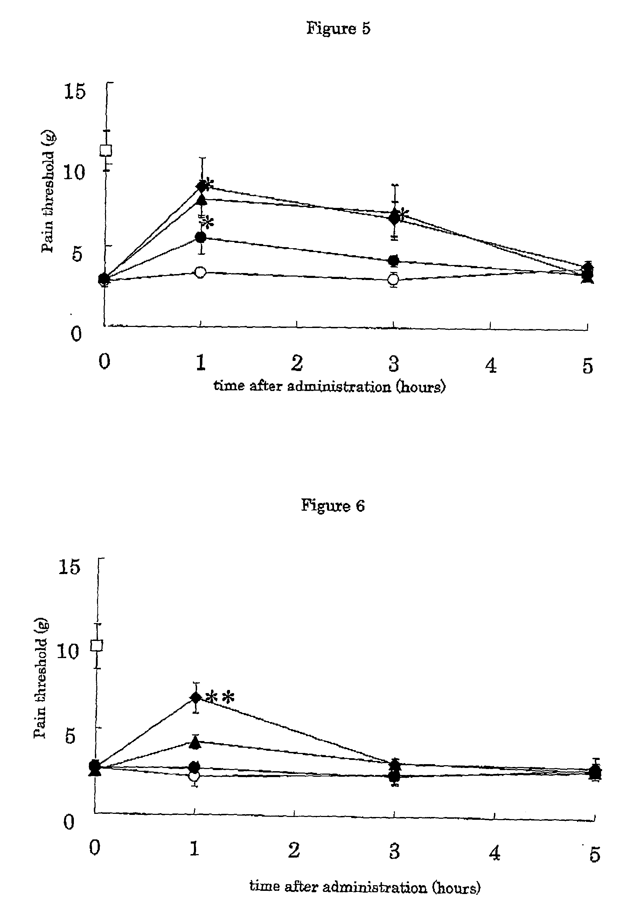

Figs. 1, 2, 3, 4, 5 and 6 show the results of thioperamide, Compound A, A-304121, A-317920, ABT-239 and JNJ-5207852 for decreases (allodynia) for pain threshold in rats with chronic constriction injury. Thioperamide, Compound A, A-304121, A-317920, ABT-239 and JNJ-5207852 improved the decreases in pain threshold which were observed in the ipsilateral paws of rats with chronic constriction injury.

### Test Example 2: Neuropathic pain inhibitory action of test compounds in rats with chronic constriction injury (2)

Rats with chronic constriction injury were prepared by the same method as in Test Example 1 and were tested day 14 to 21 after the surgery.

Cold allodynia was evaluated according to the method of Choi et al. (Pain, 1994, Vol. 59, pp. 369-376). Rats with chronic constriction injury were placed in acrylic cages (width 900 mm × length 210 mm × height 140 mm) fitted with a wire mesh floor and allowed to acclimate for at least 20 minutes. Then, about 30 µL of acetone was applied to the planter surface of the left hindpaw (near the heel) of each rat and avoidance behaviors (licking, biting, or shaking of the paw) were measured. Acetone was applied five times every 5 minutes, and rats with response frequency more than 4 times were considered as allodynia and test compound was administered. As well as in Test Example 1, each test compound was suspended in distilled water and intraperitoneally administered in a volume of 2 mL/kg.

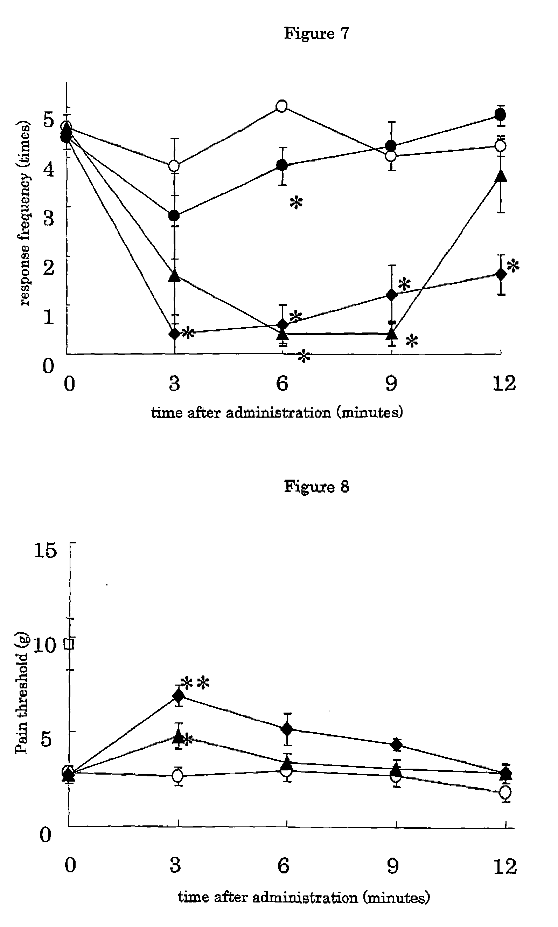

Fig. 7 shows the result of Compound A for cold allodynia in rats with chronic constriction injury. Compound A decreased responses frequency and improved the cold allodynia observed in rats with chronic constriction injury.

### Test Example 3: Neuropathic pain inhibitory action of test compounds in rats with streptozotocin-induced diabetic pain

Experiments were conducted according to the method of N. A. Calcutt et al. (British Journal of Pharmacology, 1997, Vol. 122, pp. 1478-1482). Male SD rats were intraperitoneally administered with streptozotocin at a dose of 60 mg/kg to prepare rats with streptozotocin-induced diabetic pain. Only rats showing blood glucose levels of 250 mg/dl or more 6 weeks after the administration and a 50% withdrawal threshold of 4 g or less in measurement using the von Frey filament were used for the experiments. Rats were placed in stainless cages (width 750 x length 210 x height 170 mm) and allowed to acclimate for at least 20 minutes, after that the pain threshold was determined. The pain thresholds in rats without administration of streptozotocin were about 10 g, but obvious decreases in pain threshold were observed in the diabetic rats administered with streptozotocin. As well as in Test Example 1, each test compound was suspended in distilled water and intraperitoneally administered in a volume of 2 mL/kg.

Figs. 8, 9 and 10 show the results of thioperamide, Compound A and A-304121 for the decreases in pain threshold of the rats with streptozotocin-induced diabetic pain. Thioperamide, Compound A and A-304121 improved the decreases in pain threshold observed in rats with streptozotocin-induced diabetic pain.

### Test Example 4: Neuropathic pain inhibitory action of test compounds in rats with chronic constriction injury (3)

Rats with chronic constriction injury were prepared by the same method as in Test Example 1 and tested by intracerebroventricular administration on day 14 to 21 after the surgery according to the following method:

The head of each rat was fixed to a brain stereotaxis apparatus under pentobarbital anesthesia. A stainless steel tube having a length of 21 mm, an outer diameter of 0.8 mm, and a stainless steel inner diameter of 0.5 mm was used as a guide cannula, and an inner cannula having an outer diameter of 0.4 mm and an inner diameter of 0.3 mm was used and adjusted so that 1 mm of the inner cannula will project from the tip of the guide cannula. The guide cannula was inserted into the left ventricle of the brain and fixed to the skull with dental acrylic cement. The rats were subjected to the experiments after a recovery period of 3 days to 1 week after the surgery. The inner cannula was connected to a micro syringe (50 µL) with a polyethylene tube, and test compounds were injected using a micro infusion pump (trade name: CMA/100, manufactured by CMA/Microdialysis) at a flow rate of 4 µL/min. Each test compound was dissolved in physiological saline and intracerebroventricularly administered in a volume of 10 µL.

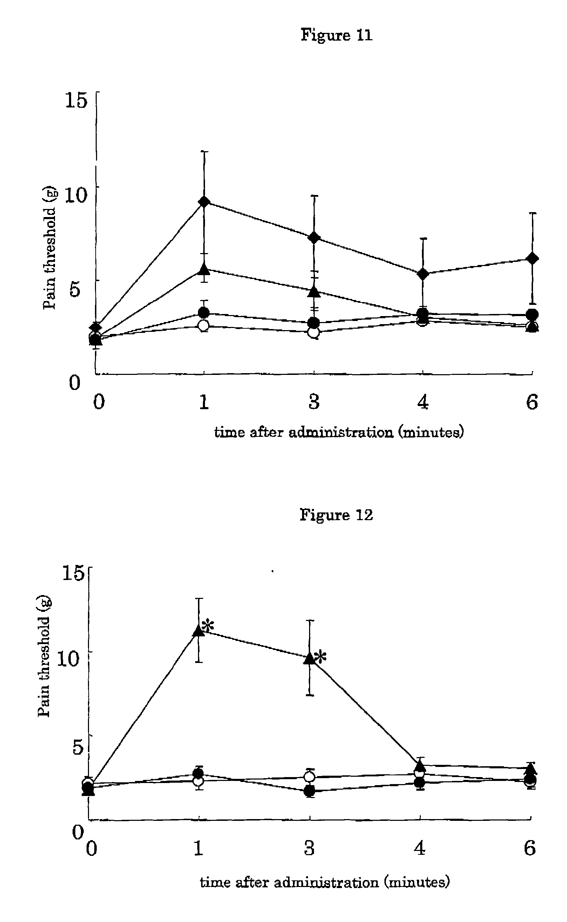

Figs. 11, 12 and 13 show the effects of clobenpropit, Compound A and A-304121 on decreased pain threshold in rats with chronic constriction injury. Clobenpropit, Compound A and A-304121 improved the decreased pain threshold observed in rats with chronic constriction injury.

### Test Example 5: Neuropathic pain inhibitory action of test compounds in rats with chronic constriction injury (4)

Antagonistic experiments were conducted to clarify whether or not the analgesic action of histamine H3-receptor antagonists is mediated by the histamine H3-receptor. Rats with chronic constriction injury were prepared by the same method as in Test Example 1 and tested by intracerebroventricular administration according to the same method as in Test Example 4. A test compound was dissolved in physiological saline and intracerebroventricularly administered in a volume of 10 µL. After the pain thresholds were measured, a vehicle (physiological saline) or the histamine H3-receptor agonist, (R)-α-methylhistamine (Nature, 1987, Vol. 327, pp. 117-123) was administered, and 15 minutes later, vehicle (physiological saline) or A-304121 was administered. The pain thresholds were assessed 15 and 30 minutes after the administration of test compounds.

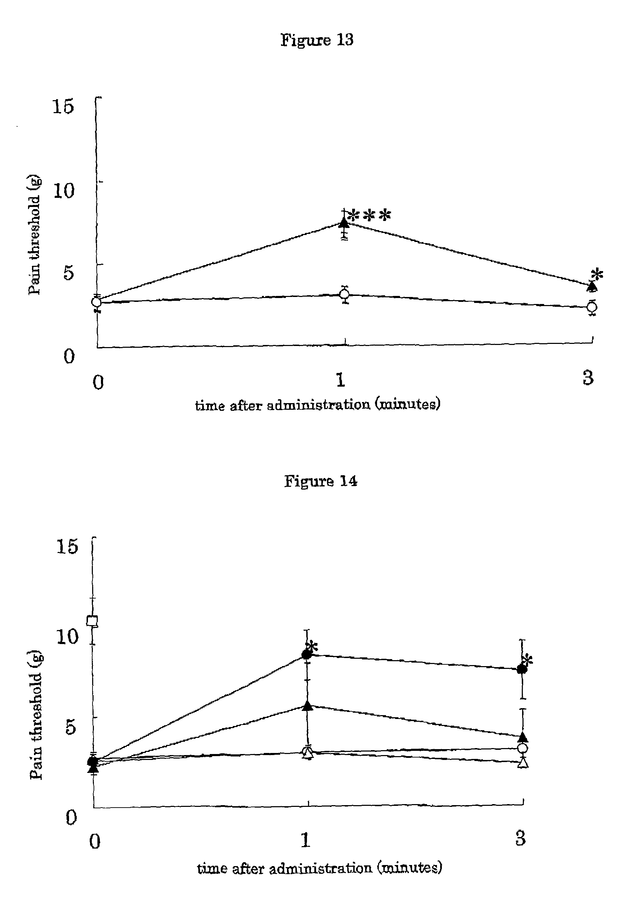

Fig. 14 shows the effects of A-304121 and (R)-α-methylhistamine on the decreases in pain threshold of rats with chronic constriction injury. A-304121 improved the decreased pain thresholds observed in rats with chronic constriction injury. On the other hand, the action of A-304121 was suppressed in the pre-treated group with (R)-α-methylhistamine, a histamine H3-receptor agonist.

These results indicate that compounds having histamine H3-receptor antagonism are effective for various models of neuropathic pain. Furthermore, the antagonism was suppressed by a histamine H3-receptor agonist.

The administration route of a medicament of the present invention is not limited, and an administration route most effective for prevention and/or treatment can be appropriately selected from oral administration and parenteral administration such as intravenous administration and the like. Examples of a formulation suitable for oral administration include tablets and the like. Examples of a formulation suitable for parenteral administration include injections and the like.

In the manufacture of solid formulations such as tablets, an excipient such as lactose or mannitol, a disintegrator such as starch, a lubricant such as magnesium stearate, a binder such as hydroxypropyl cellulose, a surfactant such as a fatty acid ester, and a plasticizer such as glycerin can be used.

A formulation suitable for parenteral administration preferably includes a sterile aqueous agent which containes an active compound and is isotonic with the blood of a recipient. For example, for an injection, a solution for injection is prepared using a carrier comprising a saline solution, a glucose solution, or a mixture of saline and a glucose solution.

The parenteral formulation may contain at least one auxiliary component selected from a diluent, a preservative, flavors, an excipient, a disintegrator, a lubricant, a binder, a surfactant, a plasticizer, and the like.

The dosage and frequency of administration of a medicament of the present invention depend on the administration form, the age and weight of a patient, and the properties and seriousness of a symptom to be treated. In oral administration, the medicament of the present invention is generally administered in an amount of 0.01 mg to 1 g/kg, preferably 0.05 to 50 mg/kg for adult, one or several times a day. However, the dosage and frequency of administration vary according to the above-described various conditions described above.

Although examples of the present invention will be described below, the present invention is not limited to these examples.

### Brief Description of the Drawings

Fig. 1 is a graph showing the effect of thioperamide on decreases in pain threshold (allodynia) of rats with chronic constriction injury, in which the pain threshold (g) is shown on the longitudinal axis, and the time (minutes) after administration is shown on the horizontal axis.
   -◆-: Group to which 100 mg/kg of thioperamide was administered.
   -▲-: Group to which 30 mg/kg of thioperamide was administered.
   -●-: Group to which 10 mg/kg of thioperamide was administered.
   -Δ-: Group to which 3 mg/kg of thioperamide was administered.
   -○-: Control
   -□-: Normal animal
   *: P < 0.05 (Dunnett-test compared with the control group)
   **: P < 0.01 (Dunnett-test compared with the control group)
Fig. 2 is a graph showing the effect of Compound A on decreases in pain threshold (allodynia) of rats with chronic constriction injury, in which the pain threshold (g) is shown on the longitudinal axis, and the time (minutes) after administration is shown on the horizontal axis.
   -◆-: Group to which 30 mg/kg of compound A was administered.
   -▲-: Group to which 10 mg/kg of compound A was administered.
   -●-: Group to which 3 mg/kg of compound A was administered.
   -○-: Control
   -□-: Normal animal
   *: P < 0.05 (Steel-test compared with the control group)
   **: P < 0.01 (Steel-test compared with the control group)
Fig. 3 is a graph showing the effect of A-304121 on decreases in pain threshold (allodynia) of rats with chronic constriction injury, in which the pain threshold (g) is shown on the longitudinal axis, and the time (minutes) after administration is shown on the horizontal axis.
   -◆-: Group to which 100 mg/kg of A-304121 was administered.
   -▲-: Group to which 30 mg/kg of A-304121 was administered.
   -●-: Group to which 10 mg/kg of A-304121 was administered.
   -○-: Control
   -□-: Normal animal
   *: P < 0.05 (Steel-test compared with the control group)
Fig. 4 is a graph showing the effect of A-317920 on decreases in pain threshold (allodynia) of rats with chronic constriction injury, in which the pain threshold (g) is shown on the longitudinal axis, and the time (minutes) after administration is shown on the horizontal axis.
   -◆-: Group to which 100 mg/kg of A-317920 was administered.
   -▲-: Group to which 30 mg/kg of A-317920 was administered.
   -●-: Group to which 10 mg/kg of A-317920 was administered.
   -○-: Control
   -□-: Normal animal
   *: P < 0.05 (Steel-test compared with the control group)
Fig. 5 is a graph showing the effect of ABT-239 on decreases in pain threshold (allodynia) of rats with chronic constriction injury, in which the pain threshold (g) is shown on the longitudinal axis, and the time (hours) after administration is shown on the horizontal axis.
   -◆-: Group to which 10 mg/kg of ABT-239 was administered.
   -▲-: Group to which 3 mg/kg of ABT-239 was administered.
   -●-: Group to which 1 mg/kg of ABT-239 was administered.
   -○-: Control
   -□-: Normal animal
   *: P < 0.05 (Steel-test compared with the control group)
Fig. 6 is a graph showing the effect of JNJ-5207852 on decreases in pain threshold (allodynia) of rats with chronic constriction injury, in which the pain threshold (g) is shown on the longitudinal axis, and the time (hours) after administration is shown on the horizontal axis.
   -◆-: Group to which 30 mg/kg of JNJ-5207852 was administered.
   -▲-: Group to which 10 mg/kg of JNJ-5207852 was administered.
   -●-: Group to which 3 mg/kg of JNJ-5207852 was administered.
   -○-: Control
   -□-: Normal animal
   *: P < 0.01 (Steel-test compared with the control group)
Fig. 7 is a graph showing the effect of compound A on stimulant-induced cold allodynia of rats with chronic constriction injury, in which the response frequency is shown on the longitudinal axis, and the time (minutes) after administration is shown on the horizontal axis.
   -◆-: Group to which 30 mg/kg of compound A was administered.
   -▲-: Group to which 10 mg/kg of compound A was administered.
   -●-: Group to which 3 mg/kg of compound A was administered.
   -○-: Control
   *: P < 0.05 (Steel-test compared with the control group)
Fig. 8 is a graph showing the effect of thioperamide on decreases in pain threshold of rats with streptozotocin-induced diabetic pain, in which the pain threshold (g) is shown on the longitudinal axis, and the time (minutes) after administration is shown on the horizontal axis.
   -◆-: Group to which 100 mg/kg of thioperamide was administered.
   -▲-: Group to which 30 mg/kg of thioperamide was administered.
   -○-: Control
   -□-: Normal animal
   *: P < 0.05 (Dunnett-test compared with the control group)
   **: P < 0.01 (Dunnett-test compared with the control group)
Fig. 9 is a graph showing the effect of Compound A on decreases in pain threshold of rats with streptozotocin-induced diabetic pain, in which the pain threshold (g) is shown on the longitudinal axis, and the time (minutes) after administration is shown on the horizontal axis.
   -◆-: Group to which 30 mg/kg of compound A was administered.
   -▲-: Group to which 10 mg/kg of compound A was administered.
   -●-: Group to which 3 mg/kg of compound A was administered.
   -○-: Control
   -□-: Normal animal
   *: P < 0.05 (Steel-test compared with the control group)
   **: P < 0.01 (Steel-test compared with the control group)

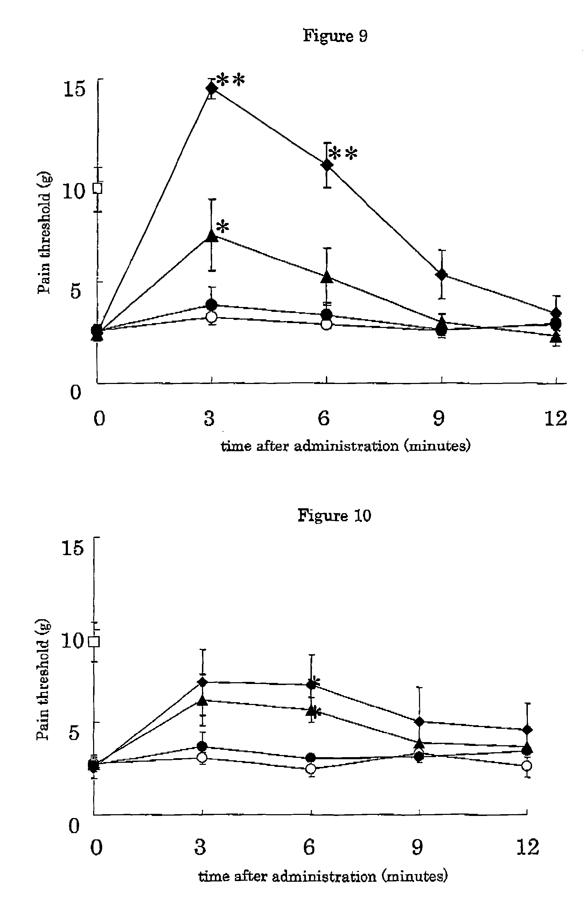

Fig. 10 is a graph showing the effect of A-304121 on decreases in pain threshold of rats with streptozotocin-induced diabetic pain, in which the pain threshold (g) is shown on the longitudinal axis, and the time (minutes) after administration is shown on the horizontal axis.
   -◆-: Group to which 100 mg/kg of A-304121 was administered.
   -▲-: Group to which 30 mg/kg of A-304121 was administered.
   -●-: Group to which 10 mg/kg of A-304121 was administered.
   -○-: Control
   -□-: Normal animal
   *: P < 0.05 (Steel-test compared with the control group)
Fig. 11 is a graph showing the effect of clobenpropit on decreases in pain threshold (allodynia) of rats with chronic constriction injury, in which the pain threshold (g) is shown on the longitudinal axis, and the time (minutes) after administration is shown on the horizontal axis.
   -◆-: Group to which 100 µg of clobenpropit was administered.
   -▲-: Group to which 30 µg of clobenpropit was administered.
   -●-: Group to which 10 µg of clobenpropit was administered.
   -○-: Control
Fig. 12 is a graph showing the effect of compound A on decreases in pain threshold (allodynia) of rats with chronic constriction injury, in which the pain threshold (g) is shown on the longitudinal axis, and the time (minutes) after administration is shown on the horizontal axis.
   -◆-: Group to which 100 µg of compound A was administered.
   -▲-: Group to which 30 µg of compound A was administered.
   -○-: Control
   *: P < 0.05 (Steel-test compared with the control group)
Fig. 13 is a graph showing the effect of A-304121 on decreases in pain threshold (allodynia) of rats with chronic constriction injury, in which the pain threshold (g) is shown on the longitudinal axis, and the time (minutes) after administration is shown on the horizontal axis.
   -◆-: Group to which 100 µg of A-304121 was administered.
   -○-: Control
   *: P < 0.05 (Steel-test compared with the control group)
   ***: P < 0.001 (Steel-test compared with the control group)
Fig. 14 is a graph showing the effects of A-304121 and (R)-(-)-α-methylhistamine on decreases in pain threshold of rats with chronic constriction injury, in which pain thresholds (g) are shown as longitudinal axis, and times (minutes) after administration are shown as horizontal axis.
   -▲-: Group to which 10 µg of (R)-(-)-α-methylhistamine and 100 µg of A-304121 were administered.
   -△-: Group to which 10 µg of (R)-(-)-α-methylhistamine and vehicle were administered.
   -●-: Group to which vehicle and 100 µg of A-304121 were administered.
   -○-: Group to which vehicle and vehicle administered.
   -□-: Normal animal
   *: P < 0.05 (Steel-test compared with the vehicle + vehicle administration group)

### Best Modes for Carryinq Out the Invention

Tablets having the following compositions are prepared according to a conventional method. Thioperamide (40 g), lactose (286.8 g) and potato starch (60 g) are mixed, then the mixture is added with a 10% aqueous solution of hydroxypropyl cellulose. The mixture is kneaded according to a conventional method, and then is granulated followed by drying and further granulation to obtain the granule for tableting. The granule is added with magnesium stearate (1.2 g) and mixed, then the resulting mixture is compressed using a tableting machine within 8 mm diameter punch and die (Type : Rt-15, Kikusui Seisakusho Ltd.) to prepare tablets each containing 20 mg of the active ingredient.

| Formulation | |
|---|---|
| Thioperamide | 20 mg |
| Lactose | 143.4 mg |
| Potato starch | 30 mg |
| Hydroxypropyl cellulose | 6 mg |
| Magnesium stearate | 0.6 mg |
| | 200 mg |

### Industrial Applicability

The present invention provides a preventive and/or therapeutic agent for neuropathic pain which comprises, as an active ingredient, a compound having histamine H3-receptor antagonism or a pharmaceutically acceptable salt thereof.

## Claims

1. A preventive and/or therapeutic agent for neuropathic pain which comprises, as an active ingredient, a compound having histamine H3-receptor antagonism or a pharmaceutically acceptable salt thereof.

2. The preventive and/or therapeutic agent for neuropathic pain according to Claim 1, wherein the compound having histamine H3-receptor antagonism is a compound represented by Formula (I): 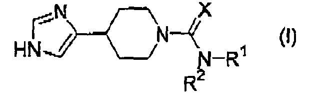
(wherein R¹ and R² may be the same or different and each represents a hydrogen atom, substituted or unsubstituted lower alkyl or substituted or unsubstituted cycloalkyl, or R¹ and R² are combined together with the adjacent nitrogen atom thereto to form a substituted or unsubstituted nitrogen containing-heterocyclic group;
and X represents an oxygen atom or a sulfur atom).

3. The preventive and/or therapeutic agent for neuropathic pain according to Claim 2, wherein one of R¹ and R² is a hydrogen atom, and the other is substituted or unsubstituted lower alkyl or substituted or unsubstituted cycloalkyl, and X is a sulfur atom.

4. The preventive and/or therapeutic agent for neuropathic pain according to Claim 1, wherein the compound having histamine H3-receptor antagonism is thioperamide.

5. The preventive and/or therapeutic agent for neuropathic pain according to Claim 1, wherein the compound having histamine H3-receptor antagonism is a compound represented by Formula (II): 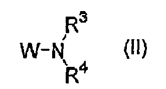
(wherein W represents a residue which imparts antagonistic and/or agonistic activity at histamine H3-receptors when attached to an imidazole ring in 4- or 5-position,
and R³ and R⁴ may be the same or different and each represents substituted or unsubstituted lower alkyl or substituted or unsubstituted cycloalkyl, or R³ and R⁴ are combined together with the adjacent nitrogen atom thereto to form a substituted or unsubstituted nitrogen containing-heterocyclic group) or a pharmaceutically acceptable salt thereof.

6. The preventive and/or therapeutic agent for neuropathic pain according to Claim 5, wherein R³ and R⁴ may be the same or different and each is substituted or unsubstituted lower alkyl or substituted or unsubstituted cycloalkyl.

7. The preventive and/or therapeutic agent for neuropathic pain according to Claim 5, wherein R³ and R⁴ are combined together with the adjacent nitrogen atom thereto to form a substituted or unsubstituted nitrogen-containing heterocyclic group.

8. The preventive and/or therapeutic agent for neuropathic pain according to Claim 5, wherein -NR³R⁴ is a group represented by Formula (III): 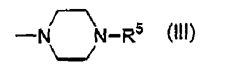
(wherein R⁵ represents lower alkyl, cycloalkyl, aryl, aralkyl, lower alkanoyl, cycloalkanoyl, aroyl, lower alkoxycarbonyl or aminoalkylcarbonyl).

9. The preventive and/or therapeutic agent for neuropathic pain according to any of Claims 5 to 8, wherein W is a group represented by Formula (IV): 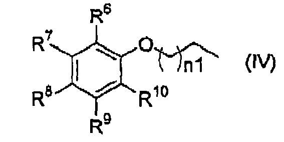
(wherein R⁶, R⁷, R⁸, R⁹ and R¹⁰ may be the same or different and each represents a hydrogen atom, halogen, amino, nitro, cyano, substituted or unsubstituted lower alkyl, substituted or unsubstituted lower alkoxy, substituted or unsubstituted lower alkanoyl, substituted or unsubstituted cycloalkanoyl, substituted or unsubstituted aroyl or substituted or unsubstituted lower alkanoylamino,
and n1 represents an integer of 1 to 7).

10. The preventive and/or therapeutic agent for neuropathic pain according to any of Claims 5 to 8, wherein W is a group represented by Formula (V): 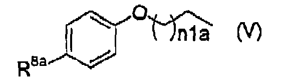
(wherein R^{8a} has the same meaning as R⁸ defined above, and nla has the same meaning as n1 defined above).

11. The preventive and/or therapeutic agent for neuropathic pain according to Claim 10, wherein n1 is 1 or 2.

12. The preventive and/or therapeutic agent for neuropathic pain according to Claim 1, wherein the compound having histamine H3-receptor antagonism is a compound represented by Formula (VI): 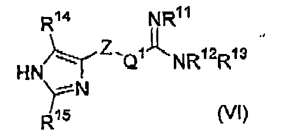
[ wherein Z represents substituted or unsubstituted lower alkylene;
Q¹ represents a sulfur atom, -NH- or -CH₂-;
R¹¹, R¹³ and R¹⁵ may be the same or different and each represents a hydrogen atom, lower alkyl, cycloalkyl, substituted or unsubstituted aryl or substituted or unsubstituted aralkyl;
R¹² represents a hydrogen atom, lower alkyl, (cycloalkyl)alkyl, substituted or unsubstituted aryl, substituted or unsubstituted aralkyl or a group represented by Formula (VII): 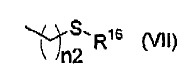
(wherein n2 represents an integer of 1 to 4, and R¹⁶ represents lower alkyl, (cycloalkyl)alkyl or substituted or unsubstituted aralkyl);
and R¹⁴ represents a hydrogen atom, halogen, amino, nitro, cyano, substituted or unsubstituted lower alkyl, substituted or unsubstituted aryl or substituted or unsubstituted aralkyl] or a pharmaceutically acceptable salt thereof.

13. The preventive and/or therapeutic agent for neuropathic pain according to Claim 1, wherein the compound having histamine H3-receptor antagonism is clobenpropit.

14. The preventive and/or therapeutic agent for neuropathic pain according to Claim 1, wherein the compound having histamine H3-receptor antagonism is a compound represented by Formula (VIII): 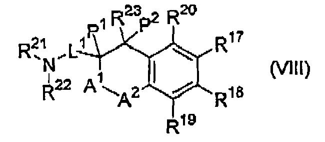
{wherein A¹ represents a bond or carbonyl;
A² represents an oxygen atom or a sulfur atom;
L¹ represents lower alkylene which may be substituted by a fluorine atom or hydroxy;
P¹ and P² represent hydrogen atoms or are combined to represent a bond;
R¹⁷, R¹⁸, R¹⁹ and R²⁰ may be the same or different and each represents a hydrogen atom, halogen, nitro, hydroxy, mercapto, cyano, carboxy, lower alkanoyl, lower alkoxycarbonyl, lower alkanoyloxy, lower alkylthio, lower alkylsulfinyl, lower alkylsulfonyl, substituted or unsubstituted lower alkyl, substituted or unsubstituted cycloalkyl, substituted or unsubstituted lower alkoxy, substituted or unsubstituted aryl, a substituted or unsubstituted heterocyclic group, -NR^{24a}R^{24b} (wherein R^{24a} and R^{24b} may be the same or different and each represents a hydrogen atom, lower alkyl or lower alkanoyl), -C(=O)-NR^{24o}R^{24d} (wherein R^{24c} and R^{24d} have the same meanings as R^{24a} and R^{24b} defined above, respectively), -SO₂-NR^{24e}R^{24f} (wherein R^{24e} and R^{24f} have the same meanings as R^{24a} and R^{24b} defined above, respectively), -L²-R²⁵ [wherein L² represents an oxygen atom, a sulfur atom, lower alkylene, lower alkenylene, -S(O)-, -S(O)₂- -C(O)-, -C(=NOR²⁶)- (wherein R²⁶ represents a hydrogen atom or lower alkyl) or -N(R²⁷)-(wherein R²⁷ represents a hydrogen atom, lower alkyl or lower alkanoyl), and R²⁵ represents cycloalkyl, aryl or a heterocyclic group], or -L³-L⁴-R²⁸ [wherein L³ represents cycloalkylene, arylene, a divalent group formed by removing any one hydrogen atom from an aliphatic heterocyclic group or heteroarylene, L⁴ represents a bond, an oxygen atom, a sulfur atom, lower alkylene, lower alkenylene, -C(O)-, -C(=NOR^{26a})- (wherein R^{26a} has the same meaning as R²⁶ defined above) or -N(R^{27a})- (wherein R^{27a} has the same meaning as R²⁷ defined above), and R²⁸ has the same meaning as R²⁵ defined above]; at least one of R¹⁷, R¹⁸, R¹⁹ and R²⁰ represents cycloalkyl, substituted or unsubstituted aryl, a substituted or unsubstituted heterocyclic group, -L²-R²⁵ (wherein L² and R²⁵ have the same meanings as defined above, respectively) or -L³-L⁴-R²⁸ (wherein L³, L⁴ and R²⁸ have the same meanings as defined above, respectively);
R²¹ and R²² may be the same or different and each represents a hydrogen atom, lower alkyl, hydroxy-lower alkyl, cycloalkyl, (cycloalkyl)alkyl, lower alkenyl, lower alkynyl, aryl, aralkyl, a heterocyclic group or heterocyclic alkyl, or R²¹ and R²² are combined together with the adjacent nitrogen atom thereto to form a substituted or unsubstituted nitrogen-containing heterocyclic group;
and R²³ represents a hydrogen atom, halogen, nitro, hydroxy, mercapto, cyano, carboxy, lower alkoxycarbonyl, lower alkanoyl, lower alkanoyloxy, lower alkylsulfinyl, lower alkylsulfonyl, lower alkylthio, aryl, a heterocyclic group, substituted or unsubstituted lower alkyl, substituted or unsubstituted lower alkoxy, -NR^{29a}R^{29b} (wherein R^{29a} and R^{29b} have the same meanings as R^{24a} and R^{24b} defined above, respectively), -C(=O)-NR^{29c}R^{29d} (wherein R^{29c} and R^{29d} have the same meanings as R^{24a} and R^{24b} defined above, respectively) or -SO₂-NR^{29e}R^{29f} (wherein R^{29e} and R^{29f} have the same meanings as R^{24a} and R^{24b} defined above, respectively)} .

15. The preventive and/or therapeutic agent for neuropathic pain according to Claim 1, wherein the compound having histamine H3-receptor antagonism is a compound represented by Formula (VIIIa):

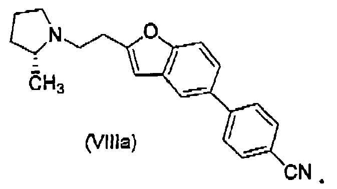

16. The preventive and/or therapeutic agent for neuropathic pain according to Claim 1, wherein the compound having histamine H3-receptor antagonism is a compound represented by Formula (IX): 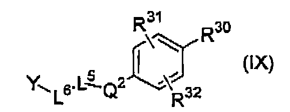
{wherein Y represents a group represented by Formula (X): 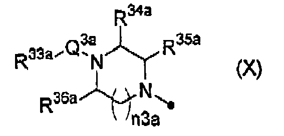
[wherein n3a is 1 or 2;
Q^{3a} represents a bond, -C(O)-, -C(S)-, -CH₂-, -SO₂- or -C(=NR³⁷)- (wherein R³⁷ represents a hydrogen atom, hydroxy, lower alkyl, cycloalkyl, (cycloalkyl)alkyl, lower alkoxy, aryl or aralkyl);
R^{33a} represents a hydrogen atom, amino, lower alkyl, lower alkoxy, cycloalkoxy, substituted or unsubstituted cycloalkyl, substituted or unsubstituted aryl, substituted or unsubstituted aryloxy, a substituted or unsubstituted heterocyclic group or -W¹-C(R^{38a}) (R^{38b})-NR^{39a}R^{39b} (wherein W¹ represents a bond or substituted or unsubstituted lower alkylene, R^{38a} and R^{38b} may be the same or different and each represents a hydrogen atom, amino, aralkyl or substituted or unsubstituted lower alkyl, and R^{39a} and R^{39b} may be the same or different and each represents a hydrogen atom, aminosulfonyl, lower alkyl, lower alkanoyl, lower alkylsulfonyl, cycloalkyl, (cycloalkyl)alkyl, cycloalkanoyl, cycloalkylsulfonyl, aralkyl, aroyl, arylsulfonyl, heterocyclic alkyl, heterocyclic carbonyl, heterocyclic alkanoyl, heterocyclic sulfonyl, substituted or unsubstituted aryl, a substituted or unsubstituted aromatic heterocyclic group or a substituted or unsubstituted aliphatic heterocyclic group, or R^{39a} and R^{39b} are combined together with the adjacent nitrogen atom thereto to form a substituted or unsubstituted nitrogen-containing heterocyclic group, or R^{38a} or R^{38b} and R^{39a} or R^{39b} are combined together with the adjacent carbon atom and nitrogen atom thereto to form a substituted or unsubstituted heterocyclic group);
and R^{34a}, R^{35a} and R^{36a} may be the same or different and each represents a hydrogen atom or lower alkyl], a group represented by Formula (XI): 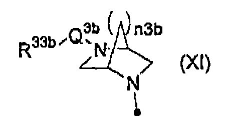
(wherein n3b, Q^{3b} and R^{33b} have the same meanings as n3a, Q^{3a} and R^{33a} defined above, respectively), a group represented by Formula (XII): 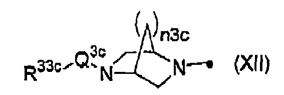
(wherein n3c, Q^{3c} and R^{33c} have the same meanings as n3a, Q^{3a} and R^{33a} defined above, respectively) or a group represented by Formula (XIII): 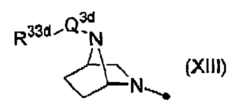
(wherein Q^{3d} and R^{33a} have the same meanings as Q^{3a} and R^{33a} defined above, respectively);
L⁵ represents a bond or lower alkylene which may be substituted by substituted or unsubstituted aryl;
L⁶ represents a bond, substituted or unsubstituted lower alkylene, or substituted or unsubstituted cycloalkylene, and L⁵ and L⁶ do not represent bonds simultaneously;
Q² represents an oxygen atom, a sulfur atom, -S(O)-, -S(O)₂- or -C≡C-;
R³⁰ represents halogen, amino, cyano, aminocarbonyl, cycloalkyl, lower alkoxy, lower alkanoyl, cycloalkanoyl, lower alkoxycarbonyl, mono- or di(lower alkyl)aminocarbonyl, aralkyl, aroyl, arylsulfonyl, aromatic heterocyclic carbonyl, aromatic heterocyclic sulfonyl, substituted or unsubstituted lower alkyl, substituted or unsubstituted aryl, a substituted or unsubstituted aromatic heterocyclic group, -CHR^{40a}-OR^{41a} (wherein R^{40a} represents a hydrogen atom, lower alkyl, cycloalkyl, (cycloalkyl)alkyl, aryl or aralkyl, and R^{41a} represents a hydrogen atom, lower alkyl, cycloalkyl, (cycloalkyl)alkyl, lower alkanoyl, lower alkoxycarbonyl, tri(lower alkyl)silyl, aryl or aralkyl), or -C(R^{40b})=N-OR^{41b} (wherein R^{40b} and R^{41b} have the same meanings as R^{40a} and R^{41a} defined above, respectively);
and R³¹ and R³² may be the same or different and each represents a hydrogen atom, halogen, amino, nitro, azido, hydroxy, cyano, formyl, carboxy, lower alkyl, perfluoro lower alkyl, lower alkenyl, lower alkynyl, lower alkoxy or perfluoro lower alkoxy, or R³¹ and R³² are combined to represent -OCH₂C(O)-}.

17. The preventive and/or therapeutic agent for neuropathic pain according to Claim 1, wherein the compound having histamine H3-receptor antagonism is a compound represented by Formula (IXa) :

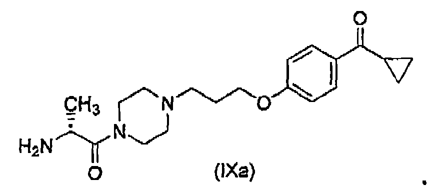

18. The preventive and/or therapeutic agent for neuropathic pain according to Claim 1, wherein the compound having histamine H3-receptor antagonism is a compound represented by Formula (IXb):

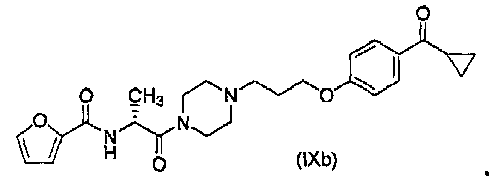

19. The preventive and/or therapeutic agent for neuropathic pain according to Claim 1, wherein the compound having histamine H3-receptor antagonism is a compound represented by Formula (XIV): 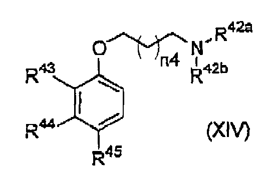
{wherein n4 represents an integer of 0 to 4;
R^{42a} and R^{42b} may be the same or different and each represents lower alkyl, lower alkenyl, cycloalkyl or (cycloalkyl)alkyl, or R^{42a} and R^{42b} are combined together with the adjacent nitrogen atom thereto to form a nitrogen-containing heterocyclic group;
and two of R⁴³, R⁴⁴ and R⁴⁵ may be the same or different and each represents a hydrogen atom or halogen, and the remainder represents a substituted or unsubstituted heterocyclic group, substituted or unsubstituted heterocyclic alkyl, substituted or unsubstituted heterocyclic alkenyl, substituted or unsubstituted heterocyclic alkynyl, -L⁷-L⁸-Q⁴ [ wherein L⁷ represents a bond or an oxygen atom, L⁸ represents substituted or unsubstituted lower alkylene, cycloalkylene, alkenylene or alkynylene, and Q⁴ represents an aliphatic heterocyclic group or -NR^{46a}R^{46b} (wherein R^{46a} and R^{46b} may be the same or different and each represents a hydrogen atom, lower alkyl, lower alkenyl, cycloalkyl, (cycloalkyl)alkyl, aryl, aralkyl, a heterocyclic group or heterocyclic alkyl)], -N(L^{8a}-Q^{4a})R⁴⁷ (wherein L^{8a} and Q^{4a} have the same meanings as L⁸ and Q⁴ defined above, respectively, and R⁴⁷ represents a hydrogen atom, lower alkyl, lower alkenyl, cycloalkyl, (cycloalkyl)alkyl, a heterocyclic group or heterocyclic alkyl) or -L⁹-C(L^{8b}-Q^{4b})R⁴⁸R⁴⁹ (wherein L^{8b} and Q^{4b} have the same meanings as L⁸ and Q⁴ defined above, respectively, L⁹ represents a bond, substituted or unsubstituted lower alkylene, cycloalkylene, alkenylene or alkynylene, R⁴⁸ represents a hydrogen atom, lower alkyl, lower alkenyl, cycloalkyl, (cycloalkyl)alkyl, a heterocyclic group or heterocyclic alkyl, and R⁴⁹ represents a hydrogen atom, halogen, hydroxy or lower alkoxy)}.

20. The preventive and/or therapeutic agent for neuropathic pain according to Claim 1, wherein the compound having histamine H3-receptor antagonism is a compound represented by Formula (XIVa):

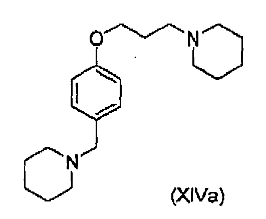

21. A method for preventing and/or treating neuropathic pain, which comprises administering an effective amount of the compound having histamine H3-receptor antagonism.

22. The method for preventing and/or treating neuropathic pain according to Claim 21, wherein the compound having histamine H3-receptor antagonism is the compound described in any of Claims 2 to 20.

23. Use of a compound having histamine H3-receptor antagonism, for the manufacture of a preventive and/or therapeutic agent for neuropathic pain.

24. The use of a compound having histamine H3-receptor antagonism according to Claim 23, wherein the compound having histamine H3-receptor antagonism is the compound described in any of Claims 2 to 20.
